# EUROPEAN PATENT APPLICATION

(11) **EP 4 389 129 A1**
(43) Date of publication of application: **26.06.2024**
(21) Application number: 22216200.0
(22) Date of filing: 22.12.2022
(51) Int. Cl.: A61K 31/568, A61P 25/14, A61P 25/16

(54) **3A-ETHYNYL-3BETA HYDROXYANDROSTAN 17-ONE OXIME FOR TREATING PARKINSON'S DISEASE AND L-DOPA-INDUCED DYSKINESIA**

(71) Applicant: Umecrine Cognition AB, 171 65 Solna (SE)
(72) Inventor: Blackburn, Thomas P., Essex, SS7 1FN (GB); Doverskog, Magnus, 114 20 Stockholm (SE); Öhman, Lars, 118 26 Stockholm (SE)
(74) Representative: Hoffmann Eitle

(57) **Abstract**

The present invention is directed to the compound golexanolone or a pharmaceutically acceptable salt thereof, for use in the treatment of Parkinson's Diseases (PD) patients, in particular PD patients exhibiting a L-dopa Induced Dyskinesia (LID).

## Description

### FIELD OF THE INVENTION

The present invention is directed to the compound golexanolone for use in the treatment of Parkinson's Disease (PD) patients, in particular PD patients exhibiting a L-dopa Induced Dyskinesia (LID).

### BACKGROUND OF THE INVENTION

Parkinson's Disease (PD) is a degenerative condition of the brain associated with motor symptoms (slow movement, tremor, rigidity, walking, and imbalance) and a wide variety of non-motor complications (fatigue, cognitive impairment, mental health disorders such as depression and anxiety, sleep disorders and pain and other sensory disturbances) (Lewitt, AP. and Chaudhuri, KR. 2020. Parkinsonism and Related Disorders, 80: S7-S12). Motor impairments, such as dyskinesias (involuntary movements) and dystonias (painful involuntary muscle contractions) contribute to limitations in speech, mobility, and restrictions in many life areas. Progression of these symptoms results in high rates of disability and care requirements. Many people with PD also develop dementia during the course of their disease (WHO 13 June 2022: Parkinson disease (who.int)*.*

While PD is the most common movement disorder, other movement disorders such as atypical parkinsonism exist such as multiple system atrophy (MSA), progressive supranuclear palsy (PSP), corticobasal degeneration (CBD), and dementia with Lewy bodies (DLB) (Przewodowska, D. et al. 2021. Frontiers in Molecular Neuroscience., August 2021, Volume 14, Article 720220*).* Some movement disorders have similar symptoms to PD such as tremor, slow movement, and rigidity. All movement disorders share the same challenges as PD regarding diagnostic and treatment gaps and access to medication, particularly in low- and middle-income countries (LMIC) (WHO 13 June 2022: Parkinson disease (who.int)*.*

Risk factors for PD include increasing age, although younger people can be affected as well. Men are more affected than women. The cause for PD is not known but is thought to arise from a complex interaction between genetic factors and exposure to environmental factors such as pesticides, solvents, and air pollution throughout life (WHO 13 June 2022: Parkinson disease (who.int).

Levo-dihydroxyphenylalanine (L-DOPA) is the most effective treatment for Parkinson's disease; however, most patients develop uncontrollable abnormal involuntary movements known as L-DOPAinduced dyskinesia (Nishijima H et al: Neurobiology of Disease 143 (2020); pp. 1-13; 104979).

Levodopa-induced dyskinesia (LID) is a form of dyskinesia associated with levodopa (L-DOPA), used to treat Parkinson's disease. It often involves hyperkinetic movements, including chorea, dystonia, and athetosis *(*Gerlach M et al: December 2011; Journal of Neural Transmission. 118 (12): pp. 1659-1660*).*

In the context of Parkinson's disease (PD), dyskinesia is often the result of long-term dopamine therapy. These motor fluctuations occur in up to 80% of PD patients after 5-10 years of L-DOPA treatment (Ahlskog JE et al 2001: Mov Disord. 16 (3): pp. 448-458), with the percentage of affected patients increasing over time *(*Obeso JA; et al. 2000; Neurology. 55 (S4): S13-S20*).*

Based on the relationship with levodopa dosing, dyskinesia most commonly occurs at the time of peak L-DOPA plasma concentrations and is thus referred to as peak-dose dyskinesia (PDD). As patients advance, they may present with symptoms of diphasic dyskinesia (DD), which occurs when the drug concentration rises or falls, and "OFF" period dystonia which occurs when the exposure of L-DOPA becomes low leading to prolonged spasms and postures (Fabbrini, A. and Guerra, A. 2021. Journal of Experimental Pharmacology, 13: 469-485). If dyskinesia becomes too severe or impairs the patient's quality of life, a reduction in L-Dopa might be necessary, however, this may be accompanied by a worsening of motor performance and "OFF" period dystonia. Therefore, once established, LID is difficult to treat (Thanvi 8 et al 2007: Postgraduate Medical Journal. 83 (980): pp. 384-388*).*

In 2017, the FDA approved amantadine (Gocovri^{®}, Adamas Pharmaceuticals) as first-line treatment for levodopa-induced dyskinesia (LID) in Parkinson's patients.

Parkinson's disease (PD) is a neurodegenerative disease that involves multiple neuronal systems-a fact readily apparent given the broad spectrum of motor and non-motor symptoms that patients with this condition develop over time (Terkelsen, MH. et al., 2022. Current Neurology and Neuroscience Reports., doi.org/10.1007/s11910-022-01245-z). However, the clinical diagnosis of PD is based on the classic motor symptoms bradykinesia, rigidity and resting tremor, caused by loss of dopaminergic projections from the substantia nigra pars compacta (SNc) to the striatum, the primary input nucleus of the motor corticostriatal circuitry. Parkinsonian symptoms are also a major feature of atypical parkinsonism, a rather heterogeneous group of neurodegenerative diseases, that can, in the beginning, be misdiagnosed as PD. Within the basal ganglia, the dopaminergic neurons from the SN pars compacta (SNc) exert modulation on the direct and indirect pathways, the two neural circuits connecting the striatum to the thalamus, decreasing the overall inhibitory output and thereby refining its processing. The inhibitory projection neurons of both these pathways, the projection medium spiny neurons and the efferents of globus pallidus, use γ-aminobutyric acid (GABA) as a transmitter. In fact, one-third of all brain synapses use GABA for inhibition.

Several reports support that enhanced GABAergic neurotransmission contribute to the pathogenesis of Parkinson's disease, and to associated motor symptoms. In particular, the report from Heo et al (Curr Biol. 2020 Jan 20; 30(2): pp. 276-291*)* shows that in animal models of Parkinson's disease the levels of GABA are increased in substantia nigra pars compacta, especially in activated astrocytes. These increased levels of GABA enhance activation of GABA_{A} receptors leading to reduced expression of tyrosine hydroxylase in neurons which, in turn, is responsible for the motor deficits in these animal models.

This effect occurs in neurons which have not yet died, and it is therefore possible to act on these mechanisms to improve motor deficits and quality of life of the patients. Heo et al (Curr Biol. 2020 Jan 20; 30(2): pp. 276-291*)* also show that inhibiting MAOb (monoamineoxidase b) reduces GABA levels and restores the expression of tyrosine hydroxylase in neurons, which is associated with improvement of motor deficits. Tyrosine hydroxylase expression is also restored by blocking GABA_{A} receptors containing the α5 subunit, supporting that enhanced activation of GABA_{A} receptors mediates the inhibition of tyrosine hydroxylase expression and motor deficits.

Other reports support that the alterations in substantia nigra pars compacta are transmitted to other brain areas, leading to alterations in cerebellum (Wu et al. Brain. 2013 Mar; 136 (Pt 3): pp. 696-709); Rusholt et al, Brain Pathol. 2020 May; 30(3): pp. 576-588), including reduced levels of tyrosine hydroxylase (Hurley et al. EurJ Neurosci. 2003 Nov;18(9): pp. 2668-2672) and appearance of alphasynuclein aggregates (Seidel et al; Ann Neurol. 2017 Jun; 81(6): pp. 898-903) which also contribute to the anxiety (Wang et al, Transl Neurosci. 2021 Oct 29; 12(1): pp. 415-424) and motor (Ballanger et al, J Neurol Neurosurg Psychiatry. 2008 Oct;79(10): pp. 1110-1116*); (*Lewis et al, Can J Neurol Sci. 2013 May; 40(3):pp. 299-306*); (*Seidel et al, Ann Neurol. 2017 Jun;81(6): pp. 898-903*)* and cognitive deficits in patients and animal models of Parkinson's disease.

In addition to the motor impairment, patients and animal models with Parkinson's disease also show cognitive impairment (Lindgren et al EurJ Neurosci. 2012 Jun;35(12): pp. 1894-1907*; (*Leao et al, 2021 Behav Brain Res. 2021 Jul 23; 410: 113349*); (*Schneider et al; Exp Neurol. 2021 Jan; 335:113514*);* and (Tian et al; Oxid Med Cell Longev. 2022 Jan 4; 2022: 2792348*).*

Likewise, the pathogenesis of LID is complex, and different neurotransmitters such as dopamine, glutamine, adenosine, and gamma-aminobutyric acid play important role altering the normal physiology of direct and indirect pathway of cortico-basal ganglia-thalamic loop responsible for fine motor control (Pandey and Srivanitchapoom. 2017. Ann Indian Acad Neurol. 20: pp. 190-198). Several reports support that enhanced GABAergic neurotransmission contributes to the pathogenesis of L-DOPA induced dyskinesia in Parkinson's disease, and to associated motor symptoms. In particular, the report from Nishijima et al. (Nishijima H et al: Neurobiology of Disease 143 (2020); pp. 1-13; 104979) showing that in animal models of Parkinson's disease the levels of GABA are increased in the medial globus pallidus, corresponding to globus pallidus interna in humans.

Nishijima et al. (Nishijima H et al: Neurobiology of Disease 143 (2020); pp. 1-13; 104979) also show that inhibiting the GABAA receptors with the experimental GABAA receptor antagonist bicuculline alleviated LID in Parkinson's disease model rats pre-treated with L-DOPA.

In summary, in accordance with the classical network model of basal ganglia, degeneration of the neurons in the SNc (substancia nigra pars compacta) leads to an imbalance of GABA and glutamate neurotransmission in the nigrostriatal system in Parkinson's disease which is associated with motor deficits and other behavioral symptoms in Parkinson's disease. This is in contrast to, for example, liver disorders such as liver cirrhosis in which motor incoordination is caused by increased levels of ammonia in the blood and inflammation leading to the impaired function of the cerebellum as described by Balzano et al (Biomedicines. 2021 Aug 12;9(8):1002. doi: 10.3390/biomedicines9081002*.*).

The compound golexanolone, having the chemical name 3α-ethynyl-3β-hydroxyandrostan-17-one oxime, is a compound currently in clinical Phase II for the treatment of Hepatic Encephalopathy (HE). This compound is disclosed in WO 2008/063128 for use in various CNS disorders. WO 2015/114308 discloses the use of the compound 3α-ethynyl-3β-hydroxyandrostan-17-one oxime for the treatment of Hepatic Encephalopathy (HE). US patent application published as US2017/0348323, discloses a method for the treatment of hypersomnolence by administering the compound 3α-ethynyl-3β-hydroxyandrostan-17-one oxime. WO 2019/102040 discloses a pharmaceutical formulation of the compound golexanolone. WO 2022/223526 discloses the compound 3α-ethynyl-3β-hydroxyandrostan-17-one oxime for use in chronic liver diseases and symptoms related thereto.

### DESCRIPTION OF THE INVENTION

An aspect of the present invention is the compound golexanolone (3α-ethynyl-3β hydroxyandrostan-17-one oxime) of formula (I) 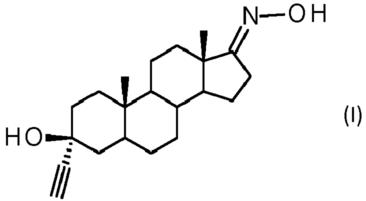 or a pharmaceutically acceptable salt thereof, for use in the treatment of Parkinson's Disease (PD) patients.

In another aspect of the present invention, the PD patients to be treated exhibit a L-dopa induced dyskinesia (LID).

A further aspect of the present invention is the compound golexanolone (3α-ethynyl-3β hydroxyandrostan-17-one oxime) of formula (I) 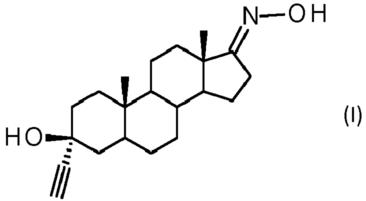 or a pharmaceutically acceptable salt thereof, for use in the treatment of L-dopa induced dyskinesia (LID) in PD patients.

### BRIEF DESCRIPTION OF DRAWINGS

Figure 1 is a schematic overview of the experimental design used herein, on rats with various symptoms of Parkinson's Disease (PD).
Figure 2 A and Figure 2B shows the test results for fatigue.
Figure 3A shows the test results for motor coordination.
Figure 3B illustrates the horizontal ladder which rats must cross in the CatWalk test.
Figure 4A, Figure 4B, and Figure 4C, shows the test results for initial dual stance in the CatWalk test.
Figure 5A and Figure 5B shows the test results for Locomotor gait swing in the catwalk test.
Figure 6 shows the test results in the object location memory test (OLM).
Figure 7 are the test results from short-term spatial memory test.
Figure 8 shows the test results for an anxiety test.
Figure 9 shows the test results for a depression test.

Throughout the figures, the following abbreviations used, has the following meaning:
SHAM VH means sham operated rats treated with vehicle; SHAM GR means sham operated rats treated with golexanolone; PARK VH means Parkinson rats treated with vehicle; and PARK GR means Parkinson rats treated with golexanolone.

### DETAILED DESCRIPTION OF THE INVENTION

An aspect of the present invention is the compound golexanolone (3α-ethynyl-3β hydroxyandrostan-17-one oxime) of formula (I) 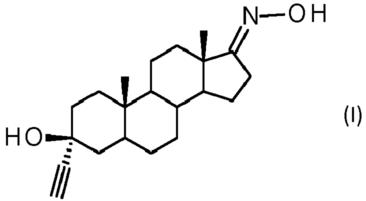 or a pharmaceutically acceptable salt thereof, for use in the treatment of Parkinson's Disease (PD) patients.

In another aspect of the present invention, the PD patients to be treated exhibit a L-dopa induced dyskinesia (LID).

A further aspect of the present invention is the compound golexanolone (3α-ethynyl-3β hydroxyandrostan-17-one oxime) of formula (I) 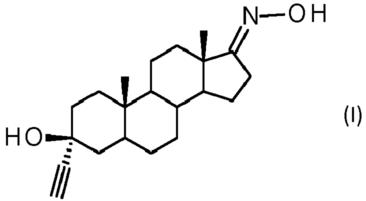 or a pharmaceutically acceptable salt thereof, for use in the treatment of L-dopa induced dyskinesia (LID) in PD patients.

An aspect of the invention is the compound golexanolone for use in the treatment of Parkinson's Disease (PD) comprising motor impairment.

An aspect of the invention is the compound golexanolone for use in the treatment of Parkinson's Disease (PD) comprising motor impairment selected from any one of, or a combination of, slow movement, tremor, posture, dexterity, gate and balance, communication skills, rigidity, walking and imbalance.

An aspect of the invention is the compound golexanolone for use in the treatment of Parkinson's Disease (PD) comprising non-motor complications.

An aspect of the invention is the compound golexanolone for use in the treatment of Parkinson's Disease (PD) comprising non-motor complications selected from any one of, or a combination of, cognitive impairment, mental health disorders such as depression or anxiety, sleep disorders, pain and sensory disturbances.

An aspect of the invention is the compound golexanolone for use in the treatment of Parkinson's Disease (PD) comprising the motor impairment dyskinesia (involuntary movement).

An aspect of the invention is the compound golexanolone for use in the treatment of Parkinson's Disease (PD) comprising the motor impairment dystonia (painful involuntary muscle contractions).

An aspect of the invention is the compound golexanolone for use in the treatment of Parkinson's Disease (PD) comprising the motor impairment slow movement or rigidity, or a combination of slow movement or rigidity.

An aspect of the invention is the compound golexanolone for use in the treatment of L-dopa Induced Dyskinesia (LID) in PD patients, comprising hyperkinetic movement.

An aspect of the invention is the compound golexanolone for use in the treatment of L-dopa Induced Dyskinesia (LID) in PD patients, comprising hyperkinetic movement selected from chorea, dystonia and athetosis, or a combination thereof.

An aspect of the invention is the compound golexanolone for use in the treatment of L-dopa Induced Dyskinesia (LID) in PD patients, which is peak-dose dyskinesia (PDD).

An aspect of the invention is the compound golexanolone for use in the treatment of L-dopa Induced Dyskinesia (LID) in PD patients, which is diphasic dyskinesia (DD).

An aspect of the invention is the compound golexanolone for use in the treatment of L-dopa Induced Dyskinesia (LID) in PD patients, which is off-period dyskinesia.

One aspect of the invention is a method for the treatment of Parkinson's Disease (PD), wherein the compound golexanolone (3α-ethynyl-3β hydroxyandrostan-17-one oxime) of formula (I) 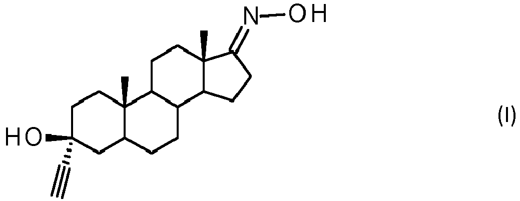 or a pharmaceutically acceptable salt thereof, is administered to a subject in need of such treatment.

In one aspect of the invention, the subject to be treated exhibits a L-dopa Induced Dyskinesia (LID).

One aspect of the invention is the use of the compound golexanolone (3α-ethynyl-3β hydroxyandrostan-17-one oxime) of formula (I) 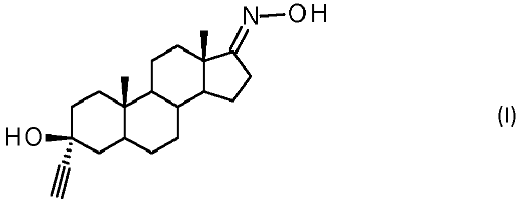 or a pharmaceutically acceptable salt thereof, for the manufacture of a medicament for the traetment of Parkinson's Disease (PD) patients.

In one aspect of the invention, the PD patients exhibit a L-dopa Induced Dyskinesia (LID).

### DEFINITIONS

As used throughout the present specification and claims, the compound golexanolone is the compound with the chemical name 3α-ethynyl-3β-hydroxyandrostan-17-one oxime, having the chemical formula (I),

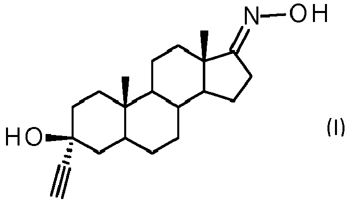

The International Nonproprietary Name (INN) is "golexanolone" and the CAS no. is 2089238-18-4.

Also within the scope of the invention is a pharmaceutically acceptable salt of the compound golexanolone, for use as described and claimed herein.

The compound golexanolone (3α-ethynyl-3β-hydroxyandrostan-17-one oxime) may be
prepared according to the method of preparation disclosed in WO 2008/063128.

The wording **Parkinson's Disease (PD)** is defined herein in accordance with the WHO definition as of 13 June 2022 (WHO 13 June 2022: Parkinson disease (who.int), and means a degenerative condition of the brain associated with **motor symptoms (motor impairments)** such as *slow movement, tremor, rigidity, walking, and imbalance,* or any combination thereof.

The wording **motor symptoms (motor impairments)** means **dyskinesias (involuntary movements)** and **dystonias (painful involuntary muscle contractions).** Motor impairment may comprise slow movement, tremor, body posture problems, dexterity problems, gate and balance problems (imbalance), communication (speech) problems, rigidity problems, or walking problems, or any combination thereof.

The wording **dyskinesias** means involuntary, erratic, writhing movements of the face, arms, legs or trunk, and may also comprise extended muscle spasms, or any combination thereof.

Dyskinesia is a complication from therapy with Parkinson's medications such as levodopa, and usually begin after a few years of treatment with **levodopa.** This is defined as **L-dopa (levodopa) Induced**

### Dyskinesia (LID).

The wording **dystonias** means **painful involuntary muscle contractions.**

Parkinson's Disease (PD) as used herein may further comprise **non-motor complications** such as *fatigue, cognitive impairment, mental health disorders such as depression and anxiety, sleep disorders and pain,* as well as other sensory disturbances as described by LeWittAP et al 2020: Parkinsonism and Related Disorders, 80: S7-512*.*

The wording **non-motor complications** as used herein is defined in accordance with LeWitt AP et al 2020: Parkinsonism and Related Disorders, 80: S7-512, and includes any one of, or a combination of, cognitive impairment, mental health disorders such as depression or anxiety, sleep disorders, pain and sensory disturbances,

The wording **L-dopa Induced Dyskinesia (LID)** comprises hyperkinetic movement. It may occur during long term duration of treatment with levodopa. LID may also be present in a patient suffering from Parkinson's Disease (PD) after as short as only a few days or months of treatment with levodopa.

**L-dopa Induced Dyskinesia (**LID) is broadly classified as ***peak-dose dyskinesia, wearing-off or off-period dyskinesia, and diphasic dyskinesia.*** Examples of hyperkinetic movement are chorea, dystonia and athetosis, or a combination thereof. The different types of Dyskenesia in L-dopa Induced Dyskinesia (LID) are as defined in Annals of Indian Academy of Neurology; Volume 20; Issue 3; July-September 2017; pp.190-198 *and* Fabrini et al: Journal of Experimental Pharmacology 2021:13 pp. 469-485*.*

The wording **peak-dose dyskinesia (PDD)** is the most common type of dyskinesia (80%), which occurs at the time of peak plasma levels of levodopa, and is characterized by stereotypic head movements, choreiform truncal movement, and ballistic limb movement, rarely myoclonus (involuntary jerky movements), can be ocular, respiratory, or abdominal muscle.

The wording **wearing-off,** also referred to as **off-period dyskinesia,** is the second most common type (30%) of L-dopa Induced Dyskinesia (LID) and typically occurs as early morning dystonia, before the first dose of levodopa, usually involves prolonged spasms and postures in the leg or feet.

The wording **diphasic dyskinesia (DD),** also referred to as **Dyskinesia-improvement-dyskinesia (DID),** is the least common (20%) and starts 10-15 min after levodopa ingestion with ipsilateral leg movement and then contralateral involvement, followed by improvement of parkinsonian symptoms for several hours and then recurrence of dyskinesia, when levodopa levels decline. This type of LID occurrs when the levodopa drug concentration in the blood rises or falls.

The wording **athetosis** means a symptom characterized by slow, involuntary, convoluted, writhing movements of the fingers, hands, toes, and feet and in some cases, arms, legs, neck and tongue.

### PHARMACEUTICAL FORMULATIONS AND ADMINISTRATION ROUTES

In certain aspects of the invention, the compound golexanolone (3α-ethynyl-3β-hydroxyandrostan-17-one oxime) as used throughout the present specification and claims, may be administered in the form of a pharmaceutical composition, in admixture with one or more pharmaceutically acceptable adjuvants, diluents and/or carriers. Examples of such pharmaceutically acceptable excipients, carriers and/or diluents useful when formulating the compound golexanolone (3α-ethynyl-3β-hydroxyandrostan-17-one oxime) for use in accordance with the present invention, are thickeners, flavoring agents, diluents, emulsifiers, dispersing aids, carrier substances, lubricants or binders. Typical pharmaceutical carriers include, but are not limited to, binding agents (e.g., pregelatinised maize starch); fillers (e.g., lactose, glucose, sucrose and other sugars, microcrystalline cellulose, pectin, gelatin, calcium sulfate, ethyl cellulose, polyacrylates or calcium hydrogen phosphate, etc.); lubricants (e.g., magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g., starch, and sodium starch glycolate); wetting agents; diluents; coloring agents; emulsifying agents; pH buffering agents; preservatives; and mixtures thereof.

In one aspect of the invention, the compound golexanolone (3α-ethynyl-3β-hydroxyandrostan-17-one oxime) may be administered by enteral administration when used as disclosed and claimed herein. Examples of enteral administration involves administration to the esophagus, stomach, and small and large intestines (i.e. the gastrointestinal tract). Methods of administration include oral, sublingual (dissolving the drug under the tongue), and rectal.

The physician will be able to determine the actual dosage of the compound golexanolone (3α-ethynyl-3β-hydroxyandrostan-17-one oxime) which will be suitable for an individual patient in order to treat Parkinson's Diseases (PD) such as L-dopa induced dyskinesia (LID). The dosage may vary with the route of administration, the severity of the disease, as well as the species, age, weight, and sex, of the patient.

In one aspect of the invention, the compound golexanolone (3α-ethynyl-3β-hydroxyandrostan-17-one oxime) may be administered as a daily dose of from 1 mg to 200 mg, 10 mg to 100 mg, 3 mg to 30 mg, 30 mg to 60 mg, 50 mg to 100 mg, 20 mg to 160 mg, 40 mg to 160 mg, or 80 mg to 160 mg.

The wording "daily dose" may be administration of the compound golexanolone (3α-ethynyl-3β-hydroxyandrostan-17-one oxime) once daily (Q.D.), or twice daily (B.I.D.). Administration twice daily (B.I.D.) means that the total daily dose is divided into two doses which in total makes up the daily dose. For example, a daily dose of 1 mg to 200 mg may be administered as a dose of 1-200 mg once daily (Q.D.), or as a dose of 0.5-100 mg twice daily (B.I.D.).

In one aspect of the invention, a daily dose of the compound golexanolone (3α-ethynyl-3β-hydroxyandrostan-17-one oxime) which may be useful in accordance with the present invention may be selected from any one of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, and 160 mg.

### EXAMPLES

### Manufacture of the compound golexanolone

The compound golexanolone (3α-ethynyl-3β-hydroxyandrostan-17-one oxime) may be
prepared according to the method of preparation disclosed in WO 2008/063128.

### BIOLOGICAL EVALUATION

### Study design

A 6-OHDA rat model as described by Heo et al (Curr Biol. 2020 Jan 20; 30(2): pp. 276-291*)* is used. Under general anesthesia, all rats receive unilateral injections of 8 µg 6-OHDA (Sigma aldrich) in 4 µL of saline with 0.1% ascorbic acid into the right medial forebrain bundle (AP -2.2 mm, L +1.5 mm relative to the bregma, and V -8.0 mm from the dura) (Paxinos G, Watson C. The Rat Brain in Stereotaxic Coordinates. 4. San Diego, USA: Academic; 1998) with the tooth bar set at +4.5 mm.

To confirm if 6-OHDA model is successfully prepared, the apomorphine (0.25 mg/kg, s.c. administration, Sigma aldrich)-induced rotation test is performed by manual counting. Inclusion criteria is above 6 rpm.

### I. Surgery: unilateral 6-OHDA lesion

The unilaterally 6-hydroxydopamine (6-OHDA) rat model according to Carvalho M et al. in Molecular Neurodegeneration 2013, 8:14; pp. 1-11*,* was used.

Eight week old Wistar-Han male rats (56 animals) (Charles River, Barcelona) were housed, two per cage, under standard laboratory conditions: 12 hour light-dark cycle, 22°C room temperature, 55% relative humidity, food and water available ad libitum. All manipulations were done in accordance with the local regulations (European Union Directive 2010/63/EU).

Under isoflurane (5% induction and then 2%) anesthesia the animals were placed on a stereotaxic frame with non-traumatic ear bars (Stoelting, USA), and unilaterally injected (left hemisphere) using an 30-gauge needle Hamilton syringe (Hamilton Company, Switzerland), with either vehicle (sham group, n = 20) or 6-OHDA hydrochloride (Sigma, USA) (6-OHDA group, n = 40) directly into the medial forebrain bundle (coordinates related to Bregma, AP = -4.4 mm; ML= -1.0 mm; DV = -7.8 mm; according to (Paxinos G, Watson C. The Rat Brain in Stereotaxic Coordinates. 4. San Diego, USA: Academic; 1998).

At a rate of 1 µl/min, sham animals received 2 µl of 0.2 mg/ml ascorbic acid in 0.9% NaCl, and 6-OHDA animals were injected with 2 µl 6-OHDA hydrochloride (4 µg/µl) with 0.2 mg/ml ascorbic acid in 0.9% NaCl. After injection the syringe was left in place for 10 minutes to allow diffusion.

### II. Experimental design to test the effect of golexanolone on symptoms of Parkinson's Disease (PD)

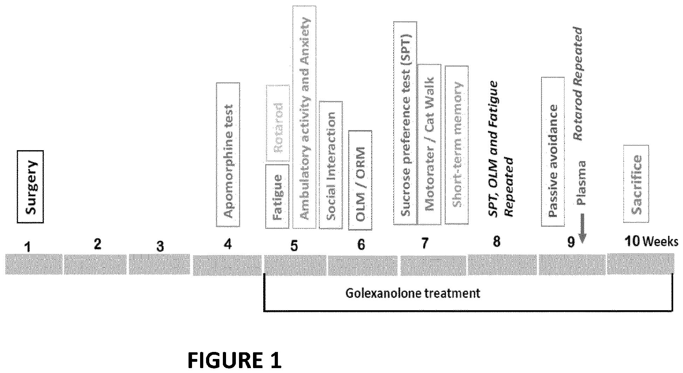

The experimental design is illustrated in **Figure 1****.**

Rats positive in the apomorphine-induced rotation test (20 animals)were included in the study, together with 20 sham-operated controls. Golexanolone treatment (50 mg/kg, daily, intragastric) and vehicle treatment as negative controls started 4 weeks after surgery. The apomorphine test is a well-known indicator of therapeutic responsiveness to dopaminergic substances and a reliable instrument for the differential diagnosis of i Parkinson's disease. The rats were divided into four groups of sham operated rats treated with vehicle; sham operated rats treated with golexanolone; Parkinson rats treated with vehicle; and Parkinson rats treated with golexanolone, respectively.

The following symptoms in rats with Parkinson's Disease were evaluated:

### Example 1

**Fatigue** was evaluated in a treadmill consisting of a motorized conveyor belt divided into two parallel sectors, each with an electrified grid. To avoid foot shocks (0.5 mV, 1 mA, 0.3Hz), the animals have to walk forward without falling off the belt. At room temperature, animals are pre-trained for one day, first exploring for 3 min without starting. Then at 10 cm/s for 5 min and then at 20 cm/s for 5 min. The test is carried out on next day. The animals are then placed on the stationary belt inclined at 5º with a gradually increasing speed of up to 30 cm/s for 5 min and continue at that speed for another 15 min (20 min total). A sensor measures the time spent on the belt. The value recorded during the last 15 minutes of each test (and the times it falls on the grid) is recorded. (Butterworth RF, Lalonde R, Power C, Baker GB, Gamrani H, Ahboucha S. Dehydroepiandrosterone sulphate improves cholestasis-associated fatigue in bile duct ligated rats. Neurogastroenterol Motil. 2009 Dec;21(12): pp. 1319-1325).

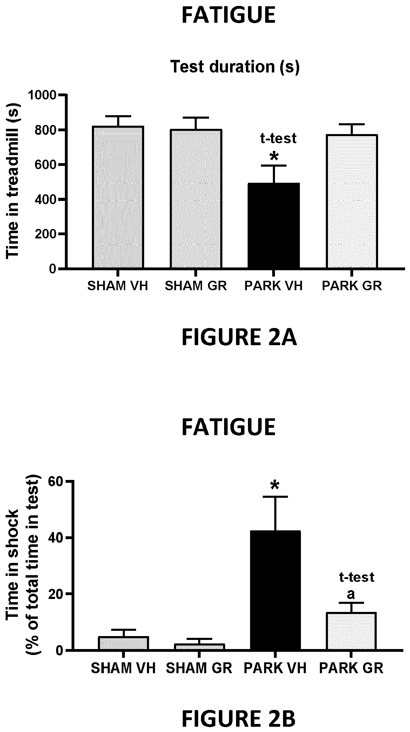

A reduction in the time in the treadmill (A) indicates increased fatigue, which is also reflected in an increase in the time in shock (B). A seen in **Figure 2** **A and** **Figure 2B****,** golexanolone improved fatigue after 5 weeks of surgery and 9 days of treatment with golexanolone.

### Example 2

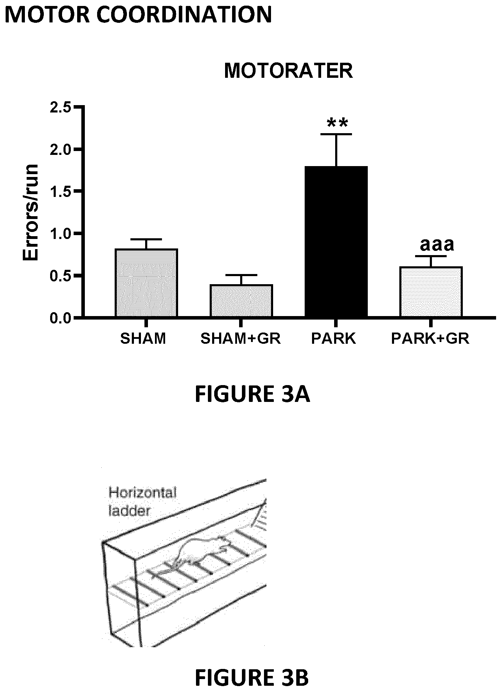

**Motor coordination** was tested with the Motorater test (Zörner B, Filli L, Starkey ML, Gonzenbach R, Kasper H, Röthlisberger M, et al. Profiling locomotor recovery: comprehensive quantification of impairments after CNS damage in rodents. Not Methods. 2010; 7(9): pp. 701-708*).* In this test, the rats must cross a horizontal ladder **(****Figure 3B****).** A kinematic analysis of locomotor performance is then conducted using the MotoRater apparatus (TSE Systems, Germany). In brief, the rats were trained to cross an illuminated glass-walled corridor to reach the dark escape box at the end. 24 hour later rats were tested to cross a ladder. The performance is recorded from the bottom by a mobile high-speed camera at 200 frames per second. Failures to set a foot on the ladder stairs are quantified. **Figure 3A** shows the effect after 7 weeks of surgery and 3 weeks of treatment of rats with Parkinson's Disease. As seen in this figure, rats treated with golexanolone, made less errors in making it across the ladder, than the control rats.

### Example 3

Motor coordination was further investigated with the CatWalk system (Lucas EK, Reid CS, McMeekin LJ, Dougherty SE, Floyd CL, Cowell RM. Cerebellar transcriptional alterations with Purkinje cell dysfunction and loss in mice locking PGC-1α. Front Cell Neurosci. 2015; 8:441*).* (Example 3 and Example 4).

The CatWalk TM system measures various aspects of locomotor pattern. Based on the position, pressure, and surface area of each footfall, multiple parameters are calculated. Trials in which the animal stopped or changed direction are excluded from subsequent analysis. Three uninterrupted trials were performed. Paw print designations were assigned and data analysed using the CatWalk analysis software (v 7.1)

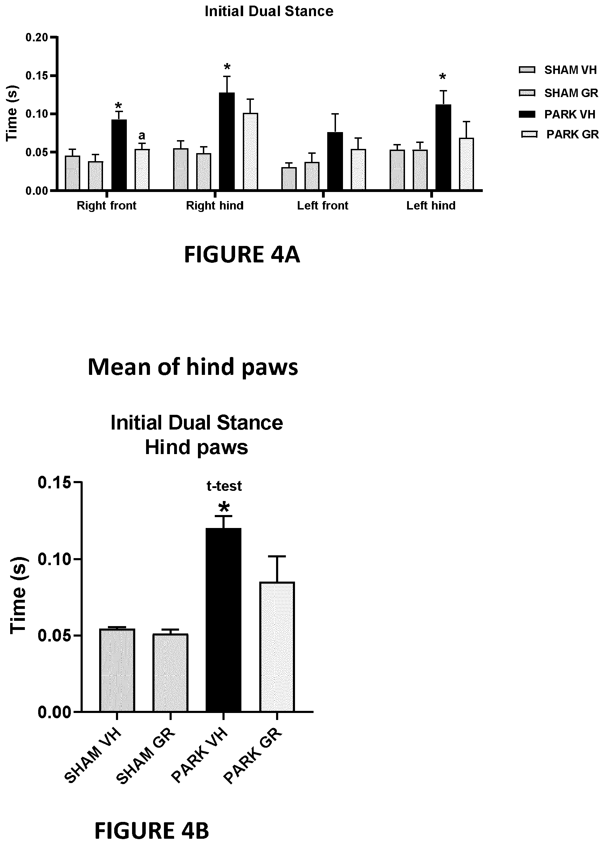

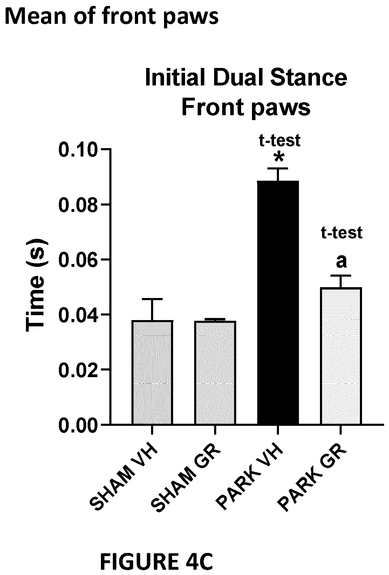

**Initial dual stance** was evaluated in the catwalk test. Initial dual distance is the duration of contact with the ground of the two hind or front paws at the same time in each step. As seen in **Figure 4A, Figure 4B****,** and **Figure 4C****,** golexanolone improves initial dual stance in the Catwalk test.

### Example 4

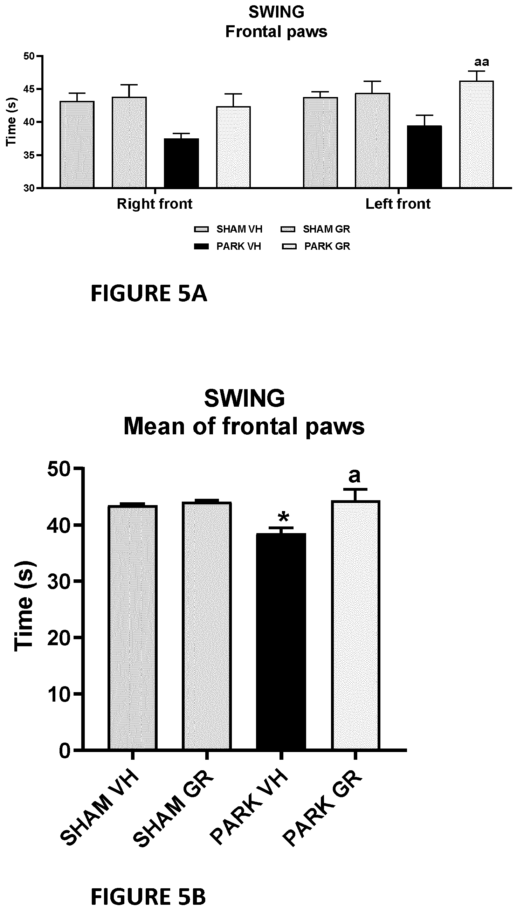

**Locomotor gait swing** was evaluated in the catwalk test. **Figure 5A** and **Figure 5B** shows the effect after 7 weeks of surgery and 3 weeks of treatment of rats with Parkinson's Disease. Swing is the time in which one paw is not in contact with the ground. As shown in Figure 5, rats treated with golexanolone improved swing. The increase in initial dual stance and the decrease in swing indicates that rats with Parkinson's keep their paws raised in each step less time than sham rats. Golexanolone improves this alteration.

### Example 5

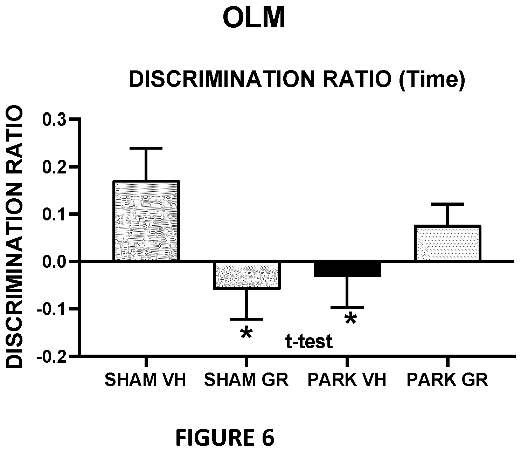

The **object location memory** test **(OLM)** evaluates **spatial location memory.** Rats are put in a field containing two identical objects. Two hours later the location of an object is changed. The time exploring each object is analyzed. The discrimination ratio is calculated: (Time exploring new object location-time exploring old located object)/ total time exploring the objects. A lower ratio indicates impaired spatial location memory. As seen from **Figure 6****,** the results are better in Parkinson's rats treated with golexanolone than in controls receving it, suggesting that golexanolone improves object location memory (OLM). This test was performed after 6 weeks of surgery and 2 weeks of treatment.

### Example 6

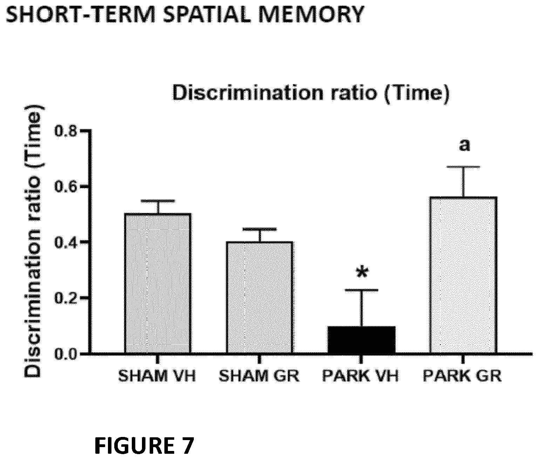

Rats explore a Y-maze with one of the three arms closed. The arm is then opened and after 1 minute the rats are placed back in the maze and should explore the newly opened arm for a longer time. If **short-term memory** is impaired, the preference for the "new" arm is reduced. We calculate a ratio: (time in the new arm-time in the old arm)/total exploration time. A reduced ratio indicates worse **short-term spatial memory.** This test was performed after 7 weeks of surgery and 3 weeks of treatment. As seen from **Figure 7****.**

### Example 7

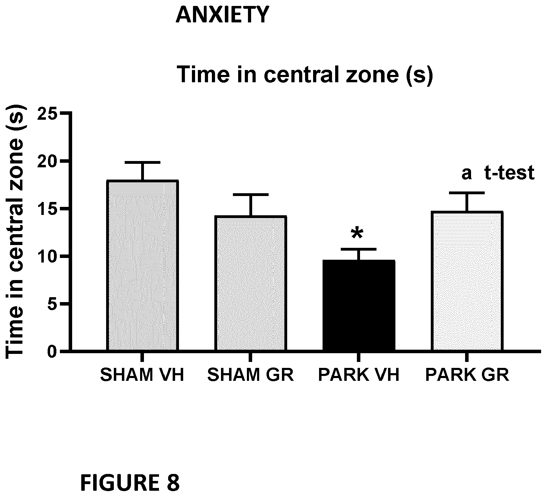

**Anxiety** was evaluated in an open arena during five minutes by measuring the time that the rat remains in the central zone, instead of close to the walls of the box, where the rats feel safer. Less time in the central zone indicates more anxiety-like behavior. This test was performed after 5 weeks of surgery and 10 days of treatment. As seen in **Figure 8****,** rats treated with golexanolone improves anxiety.

### Example 8

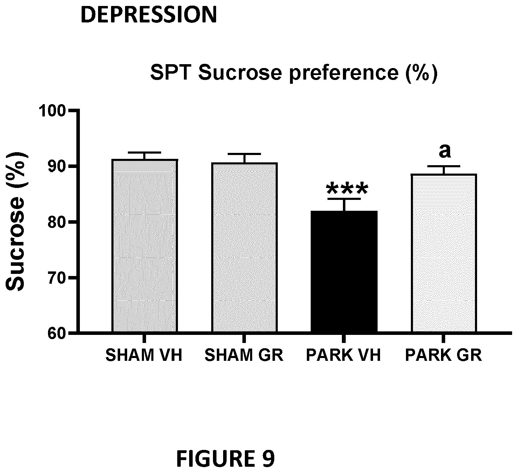

This test was performed after 7 weeks of surgery and 3 weeks of treatment. The sucrose preference test evaluates anhedonia, a symptom of depression. The test consists in measuring the preference for drinking a solution with 1% sucrose compared with water. It is performed after depriving rats of food and drink overnight. The percentage of ml of sucrose solution drunk from the total ml of liquid drunk in two hours, is represented. Reduced preference for the sucrose solution indicates anhedonia (depression) in the rats. As seen in Figure 9, rats treated with golexanolone improves depression.

## Claims

1. The compound golexanolone (3α-ethynyl-3β hydroxyandrostan-17-one oxime) of formula (I) 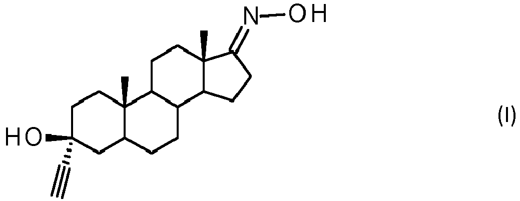 or a pharmaceutically acceptable salt thereof, for use in the treatment of Parkinson's Disease (PD) patients.

2. The compound golexanolone for use according to claim 1, wherein the Parkinson's Disease (PD) patients exhibit a L-dopa Induced Dyskinesia (LID).

3. The compound golexanolone (3α-ethynyl-3β hydroxyandrostan-17-one oxime) of formula (I) 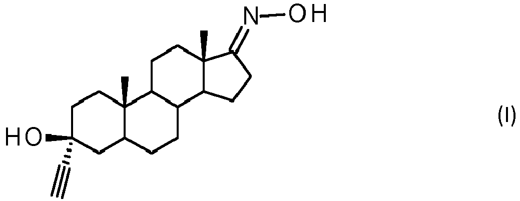 or a pharmaceutically acceptable salt thereof, for use in the treatment of L-dopa Induced Dyskinesia (LID) in Parkinson's Disease (PD) patients.

4. The compound golexanolone for use according to any one of claims 1 to 3, wherein the Parkinson's Disease (PD) comprises motor impairment.

5. The compound golexanolone for use according to claim 4, wherein the motor impairment is selected from any one of, or a combination of, slow movement, tremor, posture, dexterity, gate and balance, communication skills, rigidity, walking and imbalance.

6. The compound golexanolone for use according to any one of claims 1 to 3, wherein the Parkinson's Disease (PD) comprises non-motor complications.

7. The compound golexanolone for use according to claim 6, wherein the non-motor complications are selected from any one of, or a combination of, cognitive impairment, mental health disorders such as depression or anxiety, sleep disorders, pain and sensory disturbances.

8. The compound golexanolone for use according to claim 4, wherein the motor impairment is dyskinesia (involuntary movement).

9. The compound golexanolone for use according to claim 4, wherein the motor impairment is dystonia (painful involuntary muscle contractions).

10. The compound golexanolone for use according to claim 4, wherein the motor impairment is slow movement and/or rigidity.

11. The compound golexanolone for use according to claim 2 or claim 3, wherein the L-dopa Induced Dyskinesia (LID) comprises hyperkinetic movement.

12. The compound golexanolone for use according to claim 11 wherein the hyperkinetic movement comprises any one or a combination of chorea, dystonia, or athetosis.

13. The compound golexanolone for use according to claim 2 or claim 3, wherein the L-dopa Induced Dyskinesia (LID) is peak-dose dyskinesia (PDD).

14. The compound golexanolone for use according to claim 2 or claim 3, wherein the L-dopa Induced Dyskinesia (LID) is diphasic dyskinesia (DD).

15. The compound golexanolone for use according to claim 2 or claim 3, wherein the L-dopa Induced Dyskinesia (LID) is off-period dyskinesia.

16. A method for the treatment of Parkinson's Disease (PD), wherein the compound golexanolone (3α-ethynyl-3β hydroxyandrostan-17-one oxime) of formula (I) 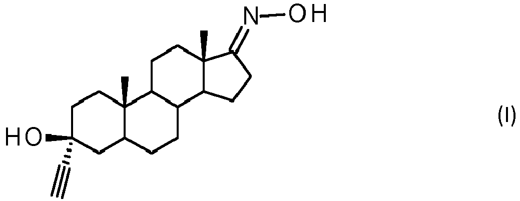 or a pharmaceutically acceptable salt thereof, is administered to a subject in need of such treatment.

17. The method of claim 16, wherein the subject exhibits a L-dopa Induced Dyskinesia (LID).

18. Use of the compound golexanolone (3α-ethynyl-3β hydroxyandrostan-17-one oxime) of formula (I) 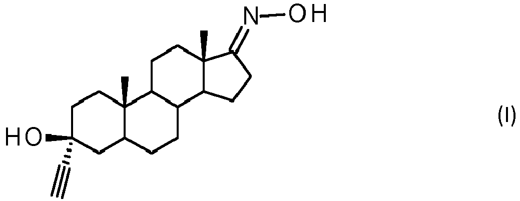 or a pharmaceutically acceptable salt thereof, for the manufacture of a medicament for the traetment of Parkinson's Disease (PD) patients.

19. The use of claim 18, wherein the PD patients exhibit a L-dopa Induced Dyskinesia (LID).
